# EUROPÄISCHE PATENTANMELDUNG

(11) **EP 3 181 163 A1**
(43) Veröffentlichungstag der Anmeldung: **21.06.2017**
(21) Anmeldenummer: 15199776.4
(22) Anmeldetag: 14.12.2015
(51) Int. Cl.: A61M 1/10

(54) **BLUTPUMPE ZUR HERZUNTERSTÜTZUNG UND VERFAHREN ZU IHREM BETRIEB**

(71) Anmelder: Berlin Heart GmbH, 12247 Berlin (DE)
(72) Erfinder: GRANEGGER, Marcus, CH-8057 Zürich (CH)
(74) Vertreter: Pfenning, Meinig & Partner mbB

(57) **Zusammenfassung**

Die Erfindung bezieht sich auf eine Blutpumpe (3) zur Herzunterstützung mit einem Rotor mit Förderelementen sowie mit einem Rotorantrieb und mit einem Drucksensor (8) und einer Regeleinrichtung (10), die mittels einer Ansteuerung des Rotorantriebs einen Druck oder einen hämodynamischen Parameter regelt, wobei insbesondere der Druck und/oder der hämodynamische Parameter mittels eines oder mehrerer hämodynamischer Sensoren und/oder aus Betriebsparametern der Pumpe (3) ermittelt werden. Durch eine derartige Regelung kann die Blutpumpe optimiert betrieben werden, und der Betrieb der Blutpumpe kann gezielt und patientenschonend variiert werden, um bestimmte Zwecke zu erreichen.

## Beschreibung

Die Erfindung liegt auf dem Gebiet der Mechanik und Elektrotechnik und ist mit besonderem Vorteil in der Medizintechnik einsetzbar.

Sie bezieht sich konkret auf Blutpumpen, wie sie zur Herzunterstützung bei menschlichen Patienten eingesetzt werden. Solche Pumpen werden eingesetzt, um die Förderung von Blut aus einem rechten oder linken Ventrikel zu unterstützen. Entsprechende Pumpen können zu diesem Zweck implantiert werden oder außen am Patientenkörper vorgesehen werden, wobei jeweils wenigstens eine Kanüle in eine Herzkammer ragt.

Derartige Pumpen werden oft als Rotationspumpen mit einem Pumpenrotor ausgebildet, der Förderelemente trägt, die das zu fördernde Blut in Radialrichtung oder Axialrichtung des Rotors transportieren. Solche Rotationspumpen haben den Vorteil, dass sich Pumpenparameter, wie beispielsweise die Fördermenge oder ein über der Pumpe aufgebauter Druck, über eine Drehzahl der Pumpe steuern bzw. regeln lassen. Auch die Variation solcher Parameter ist über eine Steuerung oder Regelung der Drehzahl einfach möglich. Mittels der Steuerung der Drehzahl kann beispielsweise über Drehzahlvariationen die Blutströmung derart verändert werden, dass ein Auswaschen der Pumpe, der Herzventrikel oder anderer Bereiche der Blutströmung erfolgt, wodurch Thrombenbildung vorgebeugt werden kann. Zudem kann die Leistung der Pumpe durch eine Änderung der Drehzahl derart gesteuert werden, dass durch die Restherzfunktion und den hierdurch erfolgenden Druckaufbau gezielt eine Aortenklappenöffnung erreicht werden kann.

Das Maß der für diese Effekte notwendigen Drehzahlvariationen ist grundsätzlich für einen individuellen Patienten nicht bekannt, so dass im Betrieb nicht sichergestellt werden kann, dass die gewollte Folge einer Drehzahlvariation eintritt.

Der vorliegenden Neuerung liegt vor dem Hintergrund des Standes der Technik die Aufgabe zugrunde, eine Blutpumpe und ein Verfahren zu ihrem Betrieb zu schaffen, bei denen durch Drehzahlvariationen verlässlich bestimmte hämodynamische Parameter einstellbar sind. Eine weitere Aufgabe besteht darin, bei Erreichen bestimmter hämodynamischer Parameter komplexere Messungen zu ermöglichen.

Die vorliegende Neuerung bezieht sich demnach auf eine Blutpumpe zur Herzunterstützung mit einem Rotor mit Förderelementen sowie mit einem Rotorantrieb und mit einem Drucksensor oder einem anderen hämodynamischen Sensor und einer Regeleinrichtung, die mittels einer Ansteuerung des Rotorantriebs einen Druck oder einen hämodynamischen Parameter regelt, wobei insbesondere der Druck und/oder der hämodynamische Parameter mittels eines oder mehrerer hämodynamischer Sensoren und/oder aus Betriebsparametern der Pumpe ermittelt werden.

Mittels des Drucksensors kann unmittelbar ein Druck gemessen werden, der beispielsweise in einem Ventrikel zu bestimmten Zeitpunkten des Herzrhythmus erreicht wird, oder auch ein Druckverlauf. Da mittels des Drucksensors der genaue Druckverlauf in einem Ventrikel periodisch aufgezeichnet werden kann, können auch andere hämodynamische Parameter aus dem periodischen Druckverlauf bestimmt werden, wie beispielsweise der enddiastolische Druck in einem Ventrikel, die Druckpulsatilität, der Druckanstieg oder -abfall dP/dt oder auch das Maximum des Druckanstiegs bzw. Druckabfalls. Auf diese Weise kann beispielsweise eine Abschätzung der Kontraktilität bestimmt werden als Verhältnis (Steigung) der Kurve, in der der enddiastolische Druck jeweils zu dem gemessenen Maximum dP/dtₘₐₓ der Druckkurve in Beziehung gesetzt wird. Auf diese Weise kann der enddiastolische Druck (EDP) durch Änderungen der Drehzahl der Pumpe variiert, die Änderung des enddiastolischen Drucks verfolgt und jeweils der Wert dP/dtₘₐₓ während dieser Variationen gemessen werden. Die Steigung dieser Kurve ist ein vorlast- und nachlastunabhängiger Kontraktilitätsindex.

Um solche Messungen gut durchführen zu können, kann die Neuerung derart ausgestaltet sein, dass der Regeleinrichtung nacheinander verschiedene Parameterwerte, insbesondere periodisch wechselnd, für den Druck oder einen hämodynamischen Parameter als Zielwert vorgegeben oder vorgebbar sind. Damit können in dem oben angeführten Beispiel nacheinander verschiedene Werte des enddiastolischen Drucks durch Drehzahlregelung der Pumpe eingestellt und die jeweiligen Werte für dP/dtₘₐₓ ermittelt werden. Dies kann auch periodisch geschehen.

Der enddiastolische Druck wird dabei jeweils aus dem zyklischen Druckverlauf im Erfassungsbereich des Drucksensors ermittelt, und in einem Regelverfahren wird die Drehzahl der Pumpe derart geregelt, dass der enddiastolischen Druck nacheinander auf verschiedene Zielwerte eingestellt wird. Diese Regelungsfolge kann periodisch wiederholt werden. Eine Regeleinrichtung innerhalb des Systems, das auch die Blutpumpe enthält, ist entsprechend geeignet, die gewünschten hämodynamischen Druckparameter zu erfassen oder zu ermitteln und gegebenenfalls eine Abweichung von einem Zielwert der Regelung der Pumpendrehzahl zugrunde zu legen.

Zudem kann vorgesehen sein, dass eine Erfassungseinrichtung zur Erfassung der Änderung des Drucks pro Zeiteinheit im Erfassungsbereich des Drucksensors und zur Ermittlung des Maximums und/oder des Minimums dieses Wertes in einem Herzzyklus vorgesehen ist. Damit wird der Wert für dP/dt sowie dessen Maximum und/oder Minimum im Verlauf eines Herzzyklus erfasst.

Zudem kann eine Verarbeitungseinrichtung vorgesehen sein, die die ermittelten Maximalwerte der Druckänderungen zu den eingestellten Ziel-Druckparametern, insbesondere dem enddiastolischen Druck, in Beziehung setzt, insbesondere einen Quotienten aus den ermittelten Maximalwerten der Druckänderungen und den eingestellten Zieldruckparametern (enddiastolischen Fülldruckwerten) ermittelt. Der genannte Quotient ergibt einen Kontraktilitätsindex, der von dem Unterstützungsgrad und der Vor- und Nachlast des jeweiligen Ventrikels unabhängig ist.

Es kann auch vorgesehen sein, dass die Verarbeitungseinrichtung dazu geeignet ist, die Steigung einer Geraden zu ermitteln, die durch den linearen Zusammenhang zwischen den ermittelten Maximalwerten der Druckänderungen und den eingestellten Ziel-Druckparametern, insbesondere dem enddiastolischen Druck, gegeben ist.

Zudem kann zur Verwirklichung der Neuerung auch vorgesehen sein, dass die Verarbeitungseinrichtung einen Quotienten aus einer Differenz von zwei oder mehreren Maximalwerten der Druckänderungen und den jeweils diesen Werten zugeordneten Ziel-Druckparametern, insbesondere dem enddiastolischen Druck, ermittelt.

Alternativ oder zusätzlich kann auch vorgesehen sein, dass eine Echokardiographieeinrichtung zur Durchführung echokardiographischer Messungen vorgesehen ist, die durch die Änderungen der Ansteuerung des Rotorantriebs getriggert oder mit diesen synchronisiert ist.

Durch eine Kopplung der Echokardiographie mit Drehzahlvariationen können gleichzeitig zu den Druckverläufen auch morphologische Parameter, wie beispielsweise der enddiastolische Durchmesser bzw. das enddiastolische Volumen einer Herzkammer, erfasst und ihre Korrelation mit Druckparametern ermittelt werden. Auch aus dem Zusammenhang zwischen dem enddiastolischen Volumen des Ventrikels und dem Maximum der Druckänderung pro Zeiteinheit dP/dtₘₐₓ ist ein Kontraktilitätsindex des jeweiligen Ventrikels zu ermitteln.

Eine weitere vorteilhafte Ausgestaltung der Neuerung kann vorsehen, dass die Regeleinrichtung dazu eingerichtet ist, während des Betriebes den Rotorantrieb auf variierende Zielwerte so zu regeln, dass jeweils wenigstens ein vorgegebenes Merkmal eines zeitlichen Verlaufes des Drucks, eines Flusses, einer Motorstromstärke oder einer Lagerposition erreicht wird. Die Betriebsarten der Regeleinrichtung beschränken sich demnach nicht darauf, bei der Pumpenansteuerung das Erreichen bestimmter Druckwerte anzustreben, sondern als Zielwert für die Regelung kann auch ein Merkmal eines Parameterverlaufs, wie beispielsweise das Erreichen eines Maximums oder eines bestimmten Wertes der ersten Ableitung oder einer Kurvenform, angestrebt werden. Entsprechende Parameter sind beispielsweise der erfasste Ventrikeldruck, ein Fluss der Blutströmung durch die Pumpe, eine Motorstromstärke, die Lagerposition eines Rotors der Pumpe oder eine aus zeitlichen Verläufen dieser Parameter abgeleitete Größe. Die entsprechenden Parameter erfahren im Verlauf des Herzzyklus ebenfalls zyklische Entwicklungen, so dass die Parameterverläufe im Zyklus der Herzaktivität wiederkehren. Dadurch wird es der Regeleinrichtung möglich, bei jedem Zyklus das Erreichen des angestrebten Zielwertes zu überprüfen und gegebenenfalls die Ansteuerung des Pumpenmotors nachzuregeln.

Dabei können die für die Regelung vorgegebenen Zielwerte auch systematisch variiert werden, um in definierter Weise sich ändernde Bedingungen beim Betrieb der Blutpumpe zu schaffen, Dies kann beispielsweise dadurch implementiert werden, dass eine Aortenklappenöffnung aufgrund eines erfassten Signalverlaufs und/oder einer hämodynamischen Größe oder eines Betriebsparameters der Pumpe nachweisbar ist. Die Aortenklappenöffnung kann beispielsweise anhand des Druckverlaufs im Ventrikel oder anhand des Verlaufs der Motorlast während eines Herzzyklus nachgewiesen werden.

Die Ansteuerung des Pumpenmotors kann beispielsweise so erfolgen, dass die Pumpenleistung so weit gesenkt wird, bis die Vorlast ausreichend ist, um durch die Restherzaktivität einen Druckaufbau zu ermöglichen, der eine Aortenklappenöffnung bewirkt. Die Erklärung hierfür liegt darin, dass bei ausreichender Pumpleistung das Blut aus dem Ventrikel regelmäßig durch die Pumpe herausgefördert wird, so dass während eines intensiven Pumpenbetriebs fallweise die Vorlast des Ventrikels nicht ausreicht, um einen Druck innerhalb des Ventrikels zu erzeugen, der zur Öffnung der Aortenklappe ausreicht.

Aus dem Stand der Technik ist es bekannt, durch regelmäßige Absenkungen der Pumpenleistung eine Aortenklappenöffnung zu erreichen. Aufgrund der fehlenden Regelung wird das Maß der Absenkung der Pumpenleistung jedoch nicht individuell angepasst, und es kann geschehen, dass die Pumpenleistung unnötig stark abgesenkt wird, so dass zwar eine Aortenklappenöffnung erfolgt, jedoch grundsätzlich die Förderung von Blut durch die Pumpe und daher auch die Perfusion der Endorgane in diesem Zeitabschnitt gering ist.

Es kann auch vorgesehen sein, dass die Regeleinrichtung einen ersten Betriebszustand aufweist, der einem ersten Betrieb der Pumpe entspricht, und einen zweiten Betriebszustand, der dazu eingerichtet ist, für ein Zeitintervall den Rotorantrieb so zu regeln, dass eine Aortenklappenöffnung aufgrund eines erfassten Signalverlaufes und/oder einer hämodynamischen Größe oder eines Betriebsparameters der Pumpe nachweisbar ist.

Dabei kann zudem vorgesehen sein, dass eine Umschalteinrichtung vorgesehen ist, die gemäß einem vorgegebenen Zeitmuster zwischen dem ersten und dem zweiten Betriebszustand umschaltet.

In dem ersten Betriebszustand findet üblicherweise eine optimierte Unterstützung der Herzaktivität des Patienten durch die Blutpumpe statt. Durch die Umschaltung in den zweiten Betrieb wird sichergestellt, dass durch die geänderte Vorlast tatsächlich eine Aortenklappenöffnung stattfindet, wobei dies an einer hämodynamischen Größe und/oder am Verlauf eines Betriebsparameters der Pumpe nachweisbar ist. Damit wird sichergestellt, dass eine Aortenklappenöffnung erfolgt, die Pumpenaktivität aber nicht über das notwendige Maß hinaus abgesenkt wird.

Die durch derartige Drehzahlvariationen gewonnenen Informationen (Kontraktilitätsindex, Aortenklappenöffnung) sind auch dazu geeignet, die Herzunterstützung durch die Blutpumpe für den Patienten individuell anzupassen und diesen beispielsweise auch von der Unterstützung durch die Herzpumpe zu entwöhnen (Weaning).

Die Neuerung kann außerdem dadurch ausgestaltet sein, dass ein Drucksensor, insbesondere ein Absolutdrucksensor oder ein Flusssensor, unmittelbar mit der Regeleinrichtung verbunden ist. Die genannten Sensortypen dienen dazu, Größen zu erfassen, die unmittelbar mit der Blutströmung zusammenhängen. Damit können hämodynamische Parameter unmittelbar gemessen werden. Unabhängig davon können verschiedene Sensoren oder Sensoreinrichtungen vorgesehen sein, die es erlauben, Betriebsparameter der Pumpe oder des Rotors zu erfassen, wie beispielsweise ein Strommesssensor, ein Spannungssensor, ein Drehzahlsensor und/oder ein Sensor, der den in axialer Richtung wirkenden Druck des Rotors auf ein Axiallager erfasst. Durch die in Axialrichtung wirkende Last auf ein Lager lässt sich beispielsweise eine durch die Pumpe erzeugte Druckdifferenz abschätzen. Der auf ein Axiallager wirkende Druck kann beispielsweise im Falle eines magnetischen Lagers durch die Überwachung der Lagerposition geschätzt werden. Bei einem geregelten magnetischen Lager kann hierzu auch der für die Lagerregelung notwendige Lagerstrom verwendet werden.

Es kann weiterhin eine Drucksensoreinrichtung mit zwei Drucksensoren vorgesehen sein, von denen ein erster in oder an der Pumpe und ein zweiter in einem mit der Pumpe nicht unmittelbar verbundenen Volumen angeordnet ist. Auf diese Weise kann eine Korrektur der Druckmessung im Ventrikel oder unmittelbar in der Pumpe bezüglicher variabler äußerer Druckverhältnisse, wie beispielsweise bezüglich des variablen Atmosphärendrucks, erfolgen.

Die Neuerung bezieht sich außer auf eine Blutpumpe der oben beschriebenen Art auch auf ein Verfahren zum Betrieb einer solchen Pumpe und sieht insbesondere vor, dass mittels einer Regeleinrichtung des Rotorantriebs ein Druck oder ein hämodynamischer Parameter intermittierend geregelt wird, wobei insbesondere der Druck und/oder der hämodynamische Parameter mittels einer Drucksensoreinrichtung und/oder hämodynamischer Sensoren und/oder aus Betriebsparametern der Pumpe ermittelt werden.

Im Folgenden wird die Neuerung anhand von Figuren einer Zeichnung gezeigt und nachfolgend erläutert. Dabei zeigt
- Fig. 1: schematisch eine Herzpumpeneinrichtung mit einer Regeleinrichtung,
- Fig. 2: schematisch in einem Diagramm den Druckverlauf während eines Herzzyklus,
- Fig. 3: zwei Diagramme mit dem Druckverlauf in einem Herzventrikel sowie der zeitlichen Ableitung dieser Größe und
- Fig. 4: ein Diagramm, in dem der enddiastolische Druck gegen dP/dtₘₐₓ aufgetragen ist.

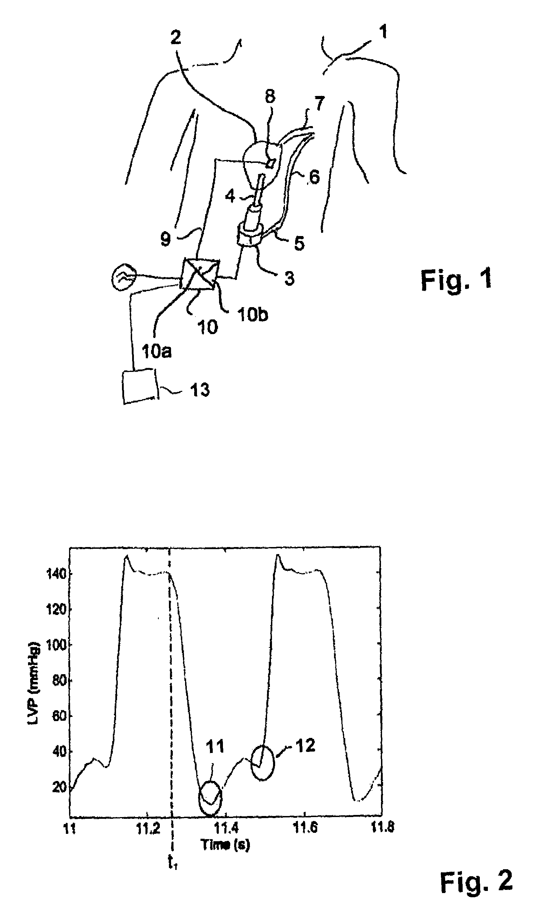

Figur 1 zeigt schematisch den Körper 1 eines Patienten sowie ein zu unterstützendes Herz 2 und eine am Herzen angeordnete Rotationspumpe 3. Die Pumpe 3 ist mittels einer Einlasskanüle 4 mit dem Herzen 2 des Patienten verbunden und saugt Blut aus dem rechten Ventrikel ab, um dieses zum Pumpenauslass 5 und durch die Auslasskanüle 6 in die Aorta 7 zu fördern. Es ist weiter ein Drucksensor 8 vorgesehen, der über eine Leitung 9 mit einer Regeleinrichtung 10 verbunden ist. Diese weist eine Speichereinrichtung 10a und eine Analyseeinrichtung 10b zur Analyse hämodynamischer Druckverläufe und zur Ableitung oder Ermittlung von Parametern hieraus auf.

In der Speichereinrichtung 10a der Regeleinrichtung 10 kann somit über einen oder mehrere Herzzyklen ein erfasster Druckverlauf erfasst und abgespeichert werden. Wie weiter unten genauer dargestellt ist, können aus dem Druckverlauf oder auch aus dem Verlauf einer anderen erfassten Größe, wie der Pumpenlast oder der Drehzahl der Pumpe, oder aus Parameterkombinationen verschiedene charakteristische hämodynamische Parameter ermittelt werden. Die Regeleinrichtung kann derart betrieben werden, dass bestimmte hämodynamische Parameterwerte erreicht werden. Die Zielwerte für die zu regelnden hämodynamischen Parameter können veränderlich sein und im Lauf der Zeit nach vorgegebenen Regeln, beispielsweise auch periodisch wiederkehrend, verändert werden. Dadurch kann die Pumpe beispielsweise auch nach einem Programm wechselweise in zwei verschiedenen Betriebszuständen betrieben werden.

Anhand der Figur 2 kann grundsätzlich der Druckverlauf im linken Ventrikel eines Herzens schematisch dargestellt werden. Die Zeit in Sekunden ist dabei auf der horizontalen Achse aufgetragen, während der Druck in Millimetern Quecksilbersäule auf der vertikalen Achse aufgetragen ist. Die Betrachtung kann zum Zeitpunkt t₁ kurz nach 11,2 Sekunden begonnen werden, wenn der Druck im linken Ventrikel abfällt, d. h. der Ventrikel relaxiert. Nach der aktiven Relaxationsphase beginnt die Füllungsphase des Ventrikels, und der Ventrikeldruck erreicht sein absolutes Minimum, das durch den ersten Markierungskreis 11 hervorgehoben ist. Füllt sich der Ventrikel wieder, so steigt der ventrikuläre Druck langsam, bis der linke Ventrikel in der Systole kontrahiert. Zum Beginn der Systole in der isovolumetrischen Kontraktionsphase steigt der Druck schlagartig an. Am Übergang zwischen dem langsamen Anstieg in der Diastole und dem schnellen Anstieg in der Systole befindet sich der enddiastolische Fülldruck, markiert durch den Kreis 12.

Bei einer Änderung der physiologischen Belastung des Patienten verändert sich sowohl der minimale diastolische Druck 11 als auch der enddiastolische Fülldruck 19. Beide genannten Werte können durch die Analyseeinrichtung 10b in der Regeleinrichtung nach Durchlaufen eines oder mehrerer Herzzyklen aus dem erfassten Druckverlauf einfach bestimmt werden. Wird durch die Regeleinrichtung die Drehzahl der Pumpe verändert, so kann hierdurch auch Einfluss auf die charakteristischen Werte, beispielsweise den enddiastolischen Fülldruck, genommen werden.

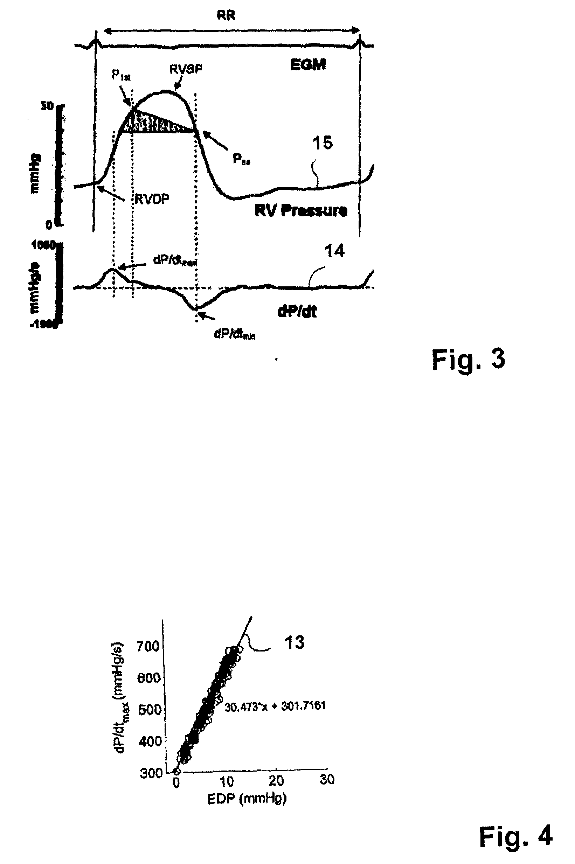

In Figur 3 sind übereinander zwei Diagramme dargestellt, wobei im oberen Diagramm für einen Herzzyklus zwischen dem Ende einer ersten Diastole und dem Ende einer zweiten Diastole der Druckverlauf 15 eines Ventrikeldrucks dargestellt ist. Der Ventrikeldruck erhöht sich dabei bis zu einem Maximum und fällt dann zum Ende der Systole ab.

Im unteren Teil der Darstellung der Figur 3 ist für denselben Zeitraum die zeitliche Ableitung dP/dt des im oberen Diagramm dargestellten Druckverlaufs gezeigt.

Es wird deutlich, dass zwei klare Extrema zu erkennen sind, zunächst auf der linken Seite der Kurve 14 der Punkt, an dem die Änderungsgeschwindigkeit dP/dt maximal ist (dP/dtₘₐₓ). Im Bereich der fallenden Flanke, also des Druckabfalls, wird ebenfalls ein Extremum in Form des Minimums dp/dtₘᵢₙ der Änderungsgeschwindigkeit erreicht. Aussagekräftig sind außer der zeitlichen Lage des Maximalwerts und des Minimalwerts der Druckänderung pro Zeiteinheit auch die absoluten Werte dP/dtₘₐₓ und dP/dtₘᵢₙ.

Eine Anwendung der Neuerung sieht vor, dass durch Änderung der Pumpendrehzahl der enddiastolische Druck (EDP) gezielt zwischen verschiedenen Werten variiert und während dieser Variationen der Wert des Maximums dP/dtₘₐₓ gemessen wird. Es ergibt sich, dass für verschiedene Werte der Pumpendrehzahl die enddiastolischen Druckwerte und die diesen jeweils zugeordneten Maxima dP/dtₘₐₓ im Diagramm (vgl. Figur 4) auf einer Geraden liegen, deren Steigung konstant ist und einen von der Vor- und Nachlast unabhängigen Kontraktilitätsindex darstellt.

In Figur 4 ist auf der horizontalen Achse der enddiastolische Fülldruck aufgetragen, während auf der vertikalen Achse die jeweils zugeordneten Werte dP/dtₘₐₓ aufgetragen sind. Die verschiedenen Messpunkte befinden sich mit guter Näherung auf einer steigenden Geraden 13.

Für die Messung ist demnach jeweils eine Regelung der Pumpe auf einen bestimmten enddiastolischen Fülldruck nötig, bei dessen Erreichen dann dP/dtₘₐₓ bestimmt wird. Ist ein solcher Messpunkt erfasst, so wird die Regelung auf einen anderen Zielwert des entsprechenden hämodynamischen Parameters, nämlich des enddiastolischen Fülldrucks, eingestellt, und es wird ein weiterer Messpunkt erfasst und so fort.

Ein weiteres Beispiel für die Anwendung der Erfindung stellt die zuverlässige Aortenklappenöffnung mittels einer Regelung der Pumpe dar. Eine Öffnung der Aortenklappe kann über bestimmte Parameter der Formkurve des gemessenen linksventrikulären Drucks oder über bestimmte veränderliche Betriebsparameter der Pumpe, wie beispielsweise den Differenzdruck und/oder einen Durchsatz, d. h. die Fördermenge pro Zeit in der Pumpe, nachgewiesen werden.

Unter bestimmten Voraussetzungen ist eine regelmäßige Öffnung der Aortenklappe wichtig. Öffnet die Aortenklappe nicht, d. h., ist die Öffnung durch Analyse der erfassten Parameter nicht nachweisbar, so wird die Pumpe nachgeregelt, beispielsweise die Pumpendrehzahl so weit weiter gesenkt, bis eine Aortenklappenöffnung eintritt.

Der Vorteil bei dieser Methode liegt darin, dass die Drehzahl nur so weit verringert wird, bis die Öffnung der Aortenklappe tatsächlich erreicht wird. Eine zu weit gehende Senkung der Drehzahl der Blutpumpe kann somit vermieden werden.

Mittels der Erfindung können somit auch beispielsweise die Kontraktilität und andere hämodynamische Parameter bei der Verwendung von LVADs (left ventricular assist devices) und RVADs (right ventricular assist devices) durch kleine, automatisch geregelte Drehzahlvariationen gemessen werden. Dadurch wird das Risiko für den Patienten gesenkt, und die Kontraktilität kann häufiger kontrolliert werden. Auch eine Erholung eines unterstützten Herzens kann auf diese Weise schnell festgestellt werden, so dass die Chancen für die Entwöhnung von der Herzunterstützung steigen.

Wird die Erfindung zum Erreichen einer regelmäßigen zuverlässigen Aortenklappenöffnung verwendet, so kann dies dazu beitragen, dass eine Aortenklappeninsuffizienz bei LVAD-Patienten verhindert wird. Durch die minimierte Drehzahlreduktion kann zudem eine mögliche kurzzeitige Unterversorgung des Patientenkörpers mit Blut vermieden werden.

## Patentansprüche

1. Blutpumpe (3) zur Herzunterstützung mit einem Rotor mit Förderelementen sowie mit einem Rotorantrieb und mit einem Drucksensor oder einem anderen hämodynamischen Sensor (8) und einer Regeleinrichtung (10), die mittels einer Ansteuerung des Rotorantriebs einen Druck oder einen hämodynamischen Parameter regelt, wobei insbesondere der Druck und/oder der hämodynamische Parameter mittels eines oder mehrerer hämodynamischer Sensoren und/oder aus Betriebsparametern der Pumpe (3) ermittelt werden.

2. Blutpumpe nach Anspruch 1, **dadurch gekennzeichnet, dass** der Regeleinrichtung (10) nacheinander verschiedene Parameterwerte, insbesondere periodisch wechselnd, für den Druck oder einen hämodynamischen Parameter als Zielwert vorgegeben oder vorgebbar sind.

3. Blutpumpe nach Anspruch 1, **dadurch gekennzeichnet, dass** die Regeleinrichtung (10) geeignet ist, einen spezifischen, insbesondere hämodynamischen Druckparameter, insbesondere den enddiastolischen Druck, ermittelt aus dem zyklischen Druckverlauf im Erfassungsbereich des Drucksensors (8), nacheinander, insbesondere periodisch, auf verschiedene Zielwerte zu regeln.

4. Blutpumpe nach Anspruch 1, 2 oder 3, **dadurch gekennzeichnet, dass** eine Erfassungseinrichtung (10a, 10b) zur Erfassung der Änderung des Drucks pro Zeiteinheit im Erfassungsbereich des Drucksensors (8) und zur Ermittlung des Maximums und/oder des Minimums dieses Wertes in einem Herzzyklus vorgesehen ist.

5. Blutpumpe nach Anspruch 1, 2, 3 oder 4, **dadurch gekennzeichnet, dass** eine Verarbeitungseinrichtung (13) vorgesehen ist, die die ermittelten Maximalwerte der Druckänderungen zu den eingestellten Ziel-Druckparametern, insbesondere dem enddiastolischen Druck, in Beziehung setzt, insbesondere einen Quotienten aus den ermittelten Maximalwerten der Druckänderungen und den eingestellten Ziel-Druckparametern ermittelt.

6. Blutpumpe nach Anspruch 1, 2, 3 oder 4, **dadurch gekennzeichnet, dass** die Verarbeitungseinrichtung (13) dazu geeignet ist, die Steigung einer Geraden zu ermitteln, die durch den linearen Zusammenhang zwischen den ermittelten Maximalwerten der Druckänderungen und den eingestellten Ziel-Druckparametern, insbesondere dem enddiastolischen Druck, gegeben ist.

7. Blutpumpe nach Anspruch 1, 2, 3 oder 4, **dadurch gekennzeichnet, dass** die Verarbeitungseinrichtung (13) einen Quotienten aus einer Differenz von zwei oder mehreren Maximalwerten der Druckänderungen und den jeweils diesen Werten zugeordneten Ziel-Druckparametern, insbesondere dem enddiastolischen Druck, ermittelt.

8. Blutpumpe nach Anspruch 1 oder 2, **dadurch gekennzeichnet, dass** eine Echokardiographieeinrichtung zur Durchführung echokardiographischer Messungen vorgesehen ist, die durch die Änderungen der Ansteuerung des Rotorantriebs getriggert oder mit diesen synchronisiert ist.

9. Blutpumpe nach Anspruch 1 oder einem der folgenden, **dadurch gekennzeichnet, dass** die Regeleinrichtung (10) dazu eingerichtet ist, während des Betriebes den Rotorantrieb auf variierende Zielwerte so zu regeln, dass jeweils wenigstens ein vorgegebenes Merkmal eines zeitlichen Verlaufs des Drucks, eines Flusses, einer Motorstromstärke oder einer Lagerposition erreicht wird.

10. Blutpumpe nach Anspruch 9, **dadurch gekennzeichnet, dass** die Regeleinrichtung (10) dazu eingerichtet ist, während des Betriebes den Rotorantrieb periodisch wechselnd so zu regeln, dass eine Aortenklappenöffnung aufgrund eines erfassten Signalverlaufs und/oder einer hämodynamischen Größe oder eines Betriebsparameters der Pumpe (3) nachweisbar ist.

11. Blutpumpe nach Anspruch 9 oder 10, **dadurch gekennzeichnet, dass** die Regeleinrichtung (10) einen ersten Betriebszustand aufweist, der einem ersten Betrieb der Pumpe (3) entspricht, und einen zweiten Betriebszustand, der dazu eingerichtet ist, für ein Zeitintervall den Rotorantrieb so zu regeln, dass eine Aortenklappenöffnung aufgrund eines erfassten Signalverlaufs und/oder einer hämodynamischen Größe oder eines Betriebsparameters der Pumpe nachweisbar ist.

12. Blutpumpe nach Anspruch 11, **dadurch gekennzeichnet, dass** eine Umschalteinrichtung vorgesehen ist, die gemäß einem vorgegebenen Zeitmuster zwischen dem ersten und dem zweiten Betriebszustand umschaltet.

13. Blutpumpe nach Anspruch 1 oder einem der folgenden, **dadurch gekennzeichnet, dass** ein Drucksensor (8), insbesondere ein Absolutdrucksensor oder ein Flusssensor, unmittelbar mit der Regeleinrichtung (10) verbunden ist.

14. Blutpumpe nach Anspruch 1 oder einem der folgenden, **dadurch gekennzeichnet, dass** eine Drucksensoreinrichtung mit zwei Drucksensoren (8) vorgesehen ist, von denen ein erster in oder an der Pumpe (3) und ein zweiter in einem mit der Pumpe nicht unmittelbar verbundenen Volumen angeordnet ist.

15. Verfahren zum Betrieb einer Blutpumpe nach Anspruch 1 oder einem der folgenden, **dadurch gekennzeichnet, dass** mittels einer Regeleinrichtung (10) des Rotorantriebs ein Druck oder ein hämodynamischer Parameter geregelt wird, wobei insbesondere der Druck und/oder der hämodynamische Parameter mittels einer Drucksensoreinrichtung (8) und/oder hämodynamischer Sensoren und/oder aus Betriebsparametern der Pumpe ermittelt werden.
